(19) Europäisches Patentamt
European Patent Office
Office européen des brevets

(11) EP 3 135 327 A1

(12) EUROPÄISCHE PATENTANMELDUNG

(43) Veröffentlichungstag:
01.03.2017 Patentblatt 2017/09

(51) Int Cl.:
A61M 1/10 (2006.01)     A61M 1/12 (2006.01)

(21) Anmeldenummer: 15183195.5

(22) Anmeldetag: 31.08.2015

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
MA

(71) Anmelder: Berlin Heart GmbH
12247 Berlin (DE)

(72) Erfinder:
• WIESENER, Constantin
  14471 Potsdam (DE)
• GRANEGGER, Marcus
  CH-8057 Zürich (CH)
• FRISCHKE, Michael
  15834 Rangsdorf (DE)

(74) Vertreter: Pfenning, Meinig & Partner mbB
Patent- und Rechtsanwälte
Joachimsthaler Straße 12
10719 Berlin (DE)

(54) PUMPE ZUR FÖRDERUNG VON FLÜSSIGKEITEN SOWIE VERFAHREN ZUR ERMITTLUNG EINER FLUSSRATE

(57) Die Neuerung bezieht sich auf eine Pumpe zur Förderung von Flüssigkeiten, insbesondere Blutpumpe, mit einer Einrichtung zur Ermittlung der Durchflussrate. Die Einrichtung zur Ermittlung der Durchflussrate kann vorteilhaft verwirklich werden durch eine in die Pumpe integrierte Einrichtung zur Zufuhr oder Ableitung von Wärmeenergie zur oder von der geförderten Flüssigkeit sowie eine in die Pumpe integrierte Einrichtung zur Ermittlung einer Temperatur, einer Temperaturänderung oder eines Temperaturgradienten.

Fig. 1

**Beschreibung**

Die Erfindung liegt auf dem Gebiet der Elektrotechnik und des Maschinenbaus und ist mit besonderem Vorteil auf dem Gebiet der Medizintechnik einsetzbar.

Konkreter bezieht sich die Erfindung auf Pumpen zur Förderung von Flüssigkeiten.

Oft ist es notwendig, im Rahmen der Förderung einer Flüssigkeit durch eine Pumpe die Flussrate zu ermitteln. Dies ist zum Beispiel durch die Erfassung des Motorstroms und einer Drehzahl und die bekannten Zusammenhänge zwischen diesen Parametern und dem Volumenstrom in einer Pumpe möglich. In einigen Fällen ist ein solches Schätzverfahren nicht genügend genau und zuverlässig, insbesondere dann, wenn sich die Viskosität verändert oder es zu einem zusätzlichen Lastmoment auf den Rotor (z.B. Thrombus) kommt.

Schließlich ist es bei Rotationspumpen mit aktiven Magnetlagern durch Überwachung der Kräfte in den geregelten Lagern möglich, ein Druckgefälle über der Pumpe zu bestimmen und hieraus unter Berücksichtigung weiterer erfasster Parameter eine Flussrate abzuschätzen.

Der vorliegenden Neuerung liegt die Aufgabe zugrunde, eine Pumpe zur Förderung von Flüssigkeiten zu schaffen, die eine möglichst zuverlässige Ermittlung der Durchflussrate erlaubt. Die Lösung der Aufgabe gelingt, insbesondere bei einer Blutpumpe, mit einer Einrichtung zur Ermittlung der Durchflussrate, wobei eine in die Pumpe integrierte Einrichtung zur Zufuhr oder Ableitung von Wärmeenergie zur oder von der geförderten Flüssigkeit sowie eine in die Pumpe integrierte Einrichtung zur Ermittlung einer Temperatur, einer Temperaturänderung oder eines Temperaturgradienten vorgesehen ist.

Wird der zu fördernden Flüssigkeit Wärmeenergie über ein Temperaturelement (Einrichtung zur Zufuhr oder Ableitung von Wärmeenergie) zugeführt oder entzogen, so ändert sich die Temperatur in Abhängigkeit von der Zeit, die die zu fördernde Flüssigkeit mit der Einrichtung zur Zufuhr oder Ableitung von Wärmeenergie in Kontakt steht, und somit in Abhängigkeit von der Durchflussrate. Die hierdurch erzielte Temperatur oder Temperaturänderung oder der hierdurch erzielte Temperaturgradient wird gemessen und zur Ermittlung einer Durchflussrate mittels bekannter Referenzwerte oder eines Kennlinienfeldes verwendet.

Die in die Pumpe integrierte Einrichtung zur Zufuhr oder Ableitung von Wärmeenergie kann beispielsweise ein Temperaturelement sein, das Wärme "erzeugt" oder "absorbiert" (das heißt selbstverständlich: eine Umwandlung zwischen Wärmeenergie und einer anderen Energieform bewirkt) und das mit der geförderten Flüssigkeit unmittelbar in Kontakt steht, beispielsweise von dieser umflossen wird. Es kann sich dabei beispielsweise um einen Heizdraht oder ein Peltier-Element handeln.

Durch eine oder mehrere Temperaturmessungen an der zu fördernden Flüssigkeit oder nahe am Temperaturelement kann eine erzeugte Temperatur oder ein Temperaturgefälle, eine Temperaturänderung oder ein Temperaturgradient ermittelt werden. Im Zusammenhang mit der Pumpe kann beispielsweise am Pumpeneinlass ein erster Temperatursensor vorgesehen sein, stromabwärts von diesem in Bezug auf die Förderrichtung der Flüssigkeit ein Temperaturelement und weiter stromabwärts, beispielsweise am Pumpenauslass, ein weiterer Temperatursensor.

Ähnlich der Thermodilutionsmethode zur Herzzeitminutenvolumenmessung kann das Temperaturelement entsprechend angesteuert werden, so dass ein reproduzierbarer Wärmeeintrag in die Flüssigkeit produziert wird. Aus der Temperatur und deren Verlauf bzw. dem Temperaturunterschied und -verlauf zwischen den am Pumpeneinlass und am Pumpenauslass gemessenen Temperaturen lässt sich dann eine Flussrate durch die Pumpe ermitteln.

Aus der US 4 465 063 ist grundsätzlich die Verwendung eines Durchflussmessers in einer Pumpe bekannt, jedoch ist dieser dort in einem Sekundärkreis eines die Pumpe antreibenden Fluids (Gases) eingebaut und steht nicht in direktem Kontakt mit der zu fördernden Flüssigkeit.

Eine besondere Ausgestaltung der Neuerung sieht vor, dass eine Einrichtung zur Zufuhr von Wärmeenergie zu der geförderten Flüssigkeit durch einen in die Pumpe integrierten Antriebsmotor gebildet ist. Die im Antriebsmotor der Pumpe umgesetzte Wärmeenergie lässt sich aus dem Motorstrom und der Spannung berechnen, so dass hierüber eine Ermittlung des Wärmeeintrags in die Flüssigkeit möglich ist. Eine Temperaturmessung stromabwärts bzw. Temperaturmessungen in der Pumpe stromaufwärts und stromabwärts der Stelle, an der der Antriebsmotor Wärme auf die geförderte Flüssigkeit übertragen kann, erlauben dann unter Berücksichtigung der Motorleistung die Ermittlung der Durchflussrate durch die Pumpe.

Es kann auch vorgesehen sein, dass eine Einrichtung zur Zufuhr von Wärmeenergie zu der geförderten Flüssigkeit durch ein oder mehrere in die Pumpe integrierte aktive, insbesondere magnetische Lager gebildet ist. Auch die Lager eines Pumpenrotors können als Wärmequellen betrachtet werden, deren Leistung ermittelbar ist, beispielsweise bei geregelten Magnetlagern durch eine Überwachung des Stroms durch die Lagerelektromagnete. Auch die durch die Lager eingetragene Wärmeenergie kann im Zusammenhang mit der ermittelten Temperatur bzw. einem ermittelten Temperaturunterschied zur Ermittlung der Durchflussrate herangezogen werden. Es kann auch die Summe aus der in den Lagern umgesetzten Leistung und der Motorantriebsleistung als Wärmeeintrag berücksichtigt werden.

Eine weitere Ausgestaltung der Neuerung kann vorsehen, dass im oder unmittelbar am Gehäuse der Pumpe ein im Strom der zu fördernden Flüssigkeit angeordnetes Fleiz-oder Kühlelement, insbesondere in Form eines Heizdrahtes oder eines Kühlelementes, insbesondere eines Peltier-Elementes, angeordnet ist. Über ein solches Heiz- oder Kühlelement, das als Heizdraht oder auch als Peltier-Element oder in einer anderen bekannten Form ausgebildet sein kann, kann ein definierter positiver oder negativer Wärmeeintrag in die strömende Flüssigkeit eingeleitet werden, und es kann die Temperaturveränderung oder ein Temperaturgradient durch entsprechende Sensoren nahe dem Temperaturelement selbst oder beispielsweise stromaufwärts und stromabwärts des Heiz- oder Kühlelementes erfasst werden. Die Funktion der Einrichtung ist in diesem Fall ähnlich wie bei einem Heizdrahtanemometer.

Es kann, wie oben bereits teilweise erläutert, zur Realisierung der Neuerung vorgesehen sein, dass eine Einrichtung zur Ermittlung der Temperatur, einer Temperaturänderung oder eines Temperaturgradienten einen ersten und/oder einen zweiten Temperatursensor umfasst, wobei einer der Temperatursensoren in der Strömung des zu fördernden Fluids stromaufwärts und der andere Temperatursensor stromabwärts der Einrichtung zur Zufuhr oder Ableitung von Wärmeenergie angeordnet ist.

Dabei kann beispielsweise auch vorgesehen sein, dass eine Einrichtung zur Ermittlung der Temperatur, einer Temperaturänderung oder eines Temperaturgradienten einen ersten und/oder einen zweiten Temperatursensor umfasst, wobei einer der Temperatursensoren in der Strömung des zu fördernden Fluids stromaufwärts eines Pumpenrotors und der andere Temperatursensor stromabwärts des Pumpenrotors angeordnet ist.

Dabei kann der Pumpenrotor auch den Antriebsmotor der Pumpe umfassen, so dass der Wärmeeintrag durch den Motor mit zu berücksichtigen ist, entweder als einziger Wärmeeintrag oder zusätzlich zu dem Wärmeeintrag durch ein Heizelement. Es kann auch ein Kühlelement mit dem Wärmeeintrag durch den Motor kombiniert werden und der Gesamtwärmesaldo durch Temperaturmessungen erfasst werden.

Es ist auch denkbar, dass eine Einrichtung zur Ermittlung der Temperatur oder einer Temperaturänderung unmittelbar an dem Heiz- oder Kühlelement angeordnet oder in dieses integriert ist. Damit wird die Temperaturmessung oder die Erfassung der Temperaturänderung oder eines Gradienten unmittelbar an der Stelle durchgeführt, an der der Wärmeeintrag oder die Abfuhr von Wärme geschieht, so dass andere Einflüsse weitgehend ausgeschaltet werden können. In dem Fall, dass die Einrichtung zur Ermittlung der Temperatur oder einer Temperaturänderung unmittelbar in das Heiz- oder Kühlelement integriert ist, kann eine physikalische Eigenschaft des Elementes, die sich mit der Temperatur ändert, direkt zur Erfassung der Temperaturgröße verwendet werden, wie beispielsweise, wenn ein temperaturabhängiger Widerstand oder ein Thermoelement in das Heiz- oder Kühlelement integriert ist.

Es kann bei der Neuerung auch vorgesehen sein, dass eine Einrichtung zur Ermittlung einer Temperaturänderung unmittelbar an dem Heiz- oder Kühlelement angeordnet ist und dass eine Regelungseinrichtung zur Einstellung einer Temperatur, einer Temperaturänderung oder eines Temperaturgradienten auf einen gegebenen Zielwert vorgesehen ist, wobei die zur Erreichung des Zielwertes zuzuführende Heiz- oder Kühlleistung geregelt wird. Damit kann durch eine Regelung auf die Erreichung einer bestimmten Temperatur oder eines bestimmten Temperaturgradienten gezielt werden, wobei durch die für die Regelung notwendige Einflussgröße, wie beispielsweise die zugeführte Energie (z. B. Strom), die Durchflussrate bestimmbar ist. Der Vorteil dieses Verfahrens bzw. dieser Vorrichtung liegt darin, dass die Arbeitstemperatur und die Temperaturdifferenz zur Umgebung (=Blut) gleich bleibt und damit temperaturabhängige Einflussgrößen auf die Bestimmung der Durchflussrate außen vor bleiben.

Es kann auch vorgesehen sein, dass im Strom der zu fördernden Flüssigkeit ein Wärme abgebender Bereich und/oder ein kühlender Bereich eines Temperaturelementes angeordnet ist und dass ein oder mehrere Temperatursensoren zur laufenden Temperaturerfassung des Elementes und/oder der vorbeiströmenden Flüssigkeit vorgesehen sind. In diesem Fall wird nicht die Temperaturentwicklung des zu fördernden Fluids herangezogen, sondern die Temperaturentwicklung des Temperaturelementes, also des Heiz- oder Kühlelementes. Dieses wird grundsätzlich durch eine elektrische Ansteuerung beheizt oder gekühlt, wobei ein Wärmetransport zum umgebenden Medium stattfindet, der von der Durchflussrate abhängig ist. Eine derartige Einrichtung lässt besonders gut die Integration des Temperaturelementes mit den Temperatursensoren zu.

Zudem kann vorgesehen sein, dass zur Erfassung der Temperatur des Temperaturelementes ein temperaturabhängiger elektrischer Widerstand oder ein Thermoelement vorgesehen ist.

Die Erfindung bezieht sich außer auf eine Pumpe der oben beschriebenen Art auch auf ein Verfahren zur Ermittlung der Flussrate einer zu fördernden Flüssigkeit durch eine Pumpe der oben beschriebenen Art, wobei die Flussrate unter Berücksichtigung einer erfassten Heiz- oder Kühlleistung, Temperatur, einer Temperaturdifferenz, einer elektrischen Motorleistung und insbesondere einer Drehzahl der Pumpe und einer elektrischen Lagerleistung ermittelt wird.

Eine besondere Ausgestaltung der Neuerung kann beispielsweise darin liegen, dass ein mit der zu fördernden Flüssigkeit in Verbindung stehender Austauschbereich eines Temperaturelementes, insbesondere eines Peltier-Elementes, abwechselnd gekühlt und geheizt wird und dass die Temperatur des Austauschbereiches laufend gemessen und aus den Temperaturänderungen und/oder Heiz- oder Kühlleistung die Flussgeschwindigkeit bestimmt wird.

Im Folgenden wird die Neuerung anhand von Ausführungsbeispielen in Figuren einer Zeichnung gezeigt und nachfolgend erläutert. Dabei zeigt

Fig. 1     in einem Längsschnitt schematisch eine Blutpumpe mit zwei markierten Positionen für Durchflusssensoren,

Fig. 2     eine Pumpe ähnlich der Ausführung in Figur 1 in einer Draufsicht,

Fig. 3     in einem Längsschnitt schematisch eine Pumpe ähnlich der in Figur 1, wobei der Antriebsmotor etwas detaillierter dargestellt ist,

Fig. 4     eine Pumpe ähnlich der in Figur 3, bei der zusätzlich eine Axiallagereinheit etwas detaillierter dargestellt ist, und

Fig. 5     schematisch den Aufbau eines beispielhaften Durchflusssensors.

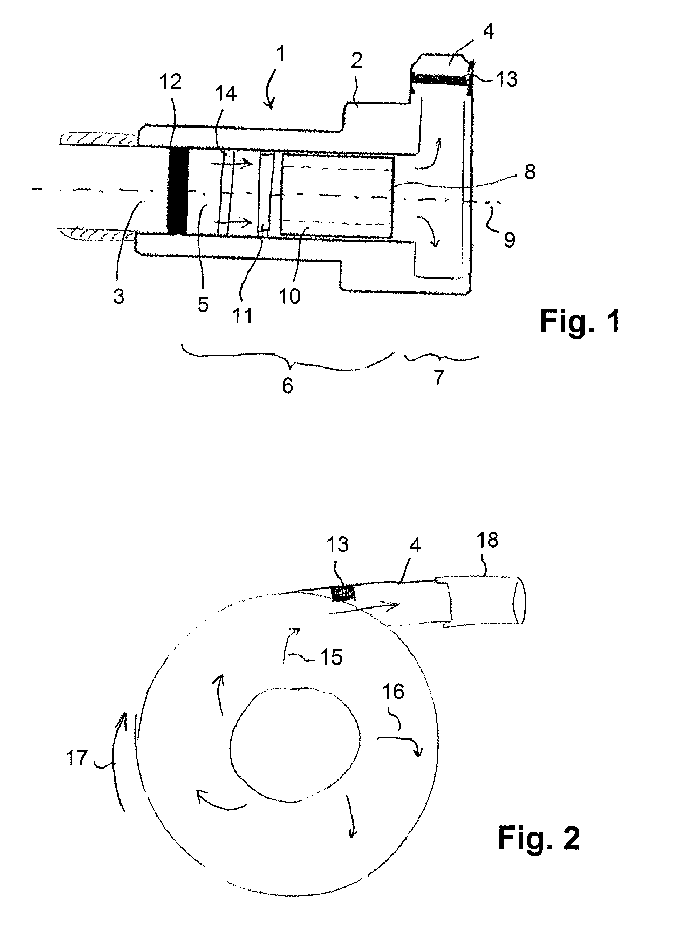

Figur 1 zeigt schematisch in einem Längsschnitt eine neuerungsgemäße Pumpe 1, die beispielsweise eine Blutpumpe zum Einsatz am lebenden Patienten sein kann. Eine solche Pumpe kann implantierbar oder teilweise implantierbar sein. Solche Pumpen können jedoch auch für andere medizinische oder nichtmedizinische Zwecke eingesetzt werden. Besondere Vorteile ergeben sich aus einer platzsparenden Integration und/oder baulichen Verbindung von einem oder mehreren Durchflusssensoren mit der Pumpe.

Eine derartige Pumpe weist typischerweise ein Pumpengehäuse 2 mit einem Pumpeneinlass 3 sowie einem Pumpenauslass 4 auf. Zwischen dem Einlass 3 und dem Auslass 4 ist ein Fluidkanal 5 vorgesehen, der bei dem gezeigten Beispiel in einem ersten Bereich 6 in axialer Richtung und in einem zweiten Bereich 7 wenigstens teilweise in radialer Richtung verläuft. Innerhalb des axialen Bereichs 6 des Fluidkanals 5 ist ein Rotor 8 vorgesehen, der um die Rotationsachse 9 antreibbar ist und der in seinem radialen Außenbereich Förderelemente 10 in Form von Förderschaufeln aufweist, die nicht im Einzelnen dargestellt sind.

Durch den Rotor 8 mit seinen Förderelementen 10 wird das Fluid, beispielsweise Blut, vom Pumpeneinlass 3 in axialer Richtung 9 gefördert, wobei das Fluid zudem einen Drall bekommt und im zweiten Bereich 7 des Fluidkanals 5 wenigstens teilweise in radialer Richtung nach außen und in Umfangsrichtung ausgelassen wird, wobei es durch Zentrifugalkräfte zu dem radial außen gelegenen Pumpenauslass gefördert wird.

Es ist festzuhalten, dass die vorliegende Neuerung jedoch auch auf rein axial fördernde Pumpen oder Pumpen mit anderen Förderprinzipien anwendbar ist.

Um die Durchflussrate der Pumpe zu bestimmen, sollten Durchflusssensoren in einem Bereich des Fluidkanals 5 angeordnet werden, in dem die Strömung möglichst homogen ist. Hierzu sind Durchflusssensoren beispielsweise direkt am Pumpeneinlass und/oder am Pumpenauslass 4 sinnvoll anzuordnen. Es wird jedoch nicht ausgeschlossen, dass derartige Durchflusssensoren auch an anderen Stellen der Pumpe, wo lokal eine Strömung stellvertretend für die Gesamtströmung durch die Pumpe erfasst werden kann, angeordnet werden können.

In Strömungsrichtung stromaufwärts von dem Rotor 8 ist in Figur 1 ein Strömungsleitrad 11 dargestellt, das Strömungsleitelemente aufweist, die der einlaufenden Strömung beispielsweise einen Drall verleihen, der die Förderleistung der Pumpe verbessert.

Der Neuerung liegt unter anderem der Gedanke zugrunde, dass die Durchflussrate einer solchen Pumpe durch lokalen Eintrag von Energie oder lokale Abfuhr von Energie und gleichzeitige Erfassung der Temperaturentwicklung gemessen werden kann, da die Entwicklung der Temperatur bei entsprechender Wärmezufuhr oder -abfuhr vom Wärmetransport durch das vorbeiströmende Blut / die vorbeiströmende Flüssigkeit bestimmt wird. Hierzu kann innerhalb der Pumpe zwischen einem ersten Temperatursensor 12 und einem zweiten Temperatursensor 13 ein Temperaturelement 14 vorgesehen sein, über das ein Wärmeenergieeintrag in die Flüssigkeit möglich ist, beispielsweise ein Heizwiderstand, der elektrisch ansteuerbar ist. Damit lässt sich die zugeführte Wärmeenergie genau bestimmen, und es kann beispielsweise mittels des ersten und zweiten Sensors 12, 13 der Temperaturunterschied bestimmt werden, der durch die Heizung mittels des Temperaturelementes 14 erzielt wird.

Besonders innerhalb einer Pumpe, in die ein Antrieb integriert ist, kann jedoch bei dem zugrunde gelegten Rechenmodell auch die Wärme berücksichtigt werden, die durch einen elektrischen Pumpenantrieb in die Flüssigkeit eingetragen wird.

In linearer Näherung ist die eingetragene Energie pro Zeiteinheit, d. h. die Wärmeleistung, proportional zum Temperaturunterschied zwischen Pumpeneinlass und Pumpenauslass. Der Proportionalitätsfaktor ist bestimmt durch ein Produkt aus der Dichte des Blutes, der spezifischen Wärmekapazität sowie der Flussrate des Blutes. Andererseits lässt sich die eingetragene Energie aus der elektrischen Leistung des Motorantriebs der Pumpe sowie, falls vorhanden, des Temperaturelements 14 berechnen.

Die entsprechende Gleichung, die das thermische Modell der Pumpe beschreibt, lautet

$$\dot{E} = \rho \cdot c \cdot Qb \left( T_{inlet} - T_{outlet} \right),$$

wobei $\dot{E}$ dem Eintrag der thermischen Energie pro Zeit entspricht, $\rho$ der Dichte des Blutes, c der spezifischen Wärmekapazität sowie $Qb$ der Flussrate des Blutes, $T_{inlet}$ der Temperatur am Pumpeneinlass und $T_{outlet}$ der Temperatur am Pumpenauslass.

Figur 2 zeigt zum näheren Verständnis des Pumpenaufbaus eine Ansicht in Axialrichtung 9 der Pumpe aus Figur 1, wobei mit Pfeilen 15, 16 die Strömungsrichtung des Blutes im Bereich 7 des Fluidkanals 5 angedeutet wird, wobei das Blut in Umfangsrichtung 17 und radial nach außen strebt und darauf zum Pumpenauslass 4 strömt. Dort ist eine Kanüle 18 angeschlossen, durch die das Blut weiter gefördert wird.

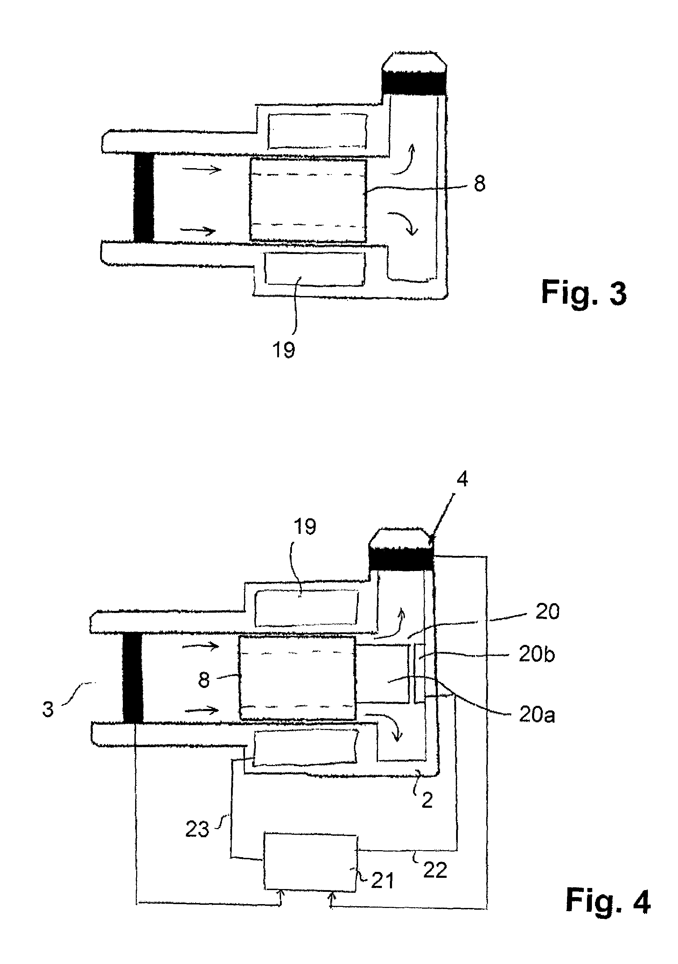

In Figur 3 ist schematisch an dem Pumpengehäuse zusätzlich zu Figur 1 ein Stator 19 eines Pumpenantriebs eingezeichnet, wobei der Stator 19 im Pumpengehäuse den Rotor 8 konzentrisch umgibt. Der Rotor 8 enthält entsprechende Magnetelemente, die zur Verwirklichung eines bürstenlosen Elektromotors dienen. Wenigstens ein Teil der Wärme, die durch den Stromfluss im Stator 19 erzeugt wird, wird in den Fluidkanal 5 eingetragen.

In Figur 4 ist zudem eine Lagereinrichtung 20 dargestellt, die einen ersten mit dem Rotor 8 rotierenden Teil 20a des Lagers sowie einen ortsfesten Teil 20b des Lagers umfasst. Der ortsfeste Teil 20b des Lagers ist Teil eines Magnetkreises, der beispielsweise über Teile des Pumpengehäuses 2 und den Stator 19 geschlossen werden kann, wobei in diesem Magnetkreis ein steuerbarer Elektromagnet integriert ist, der die magnetischen Kräfte in Axialrichtung steuern bzw. regeln kann, um eine Axiallagerung des Rotors 8 zu bewirken. Die hierfür erforderliche elektrische Leistung kann ebenfalls in die Berechnung der Wärmezufuhr, d. h. in die Berechnung des gesamten thermischen Energieeintrags in das die Pumpe durchströmende Blut einbezogen werden.

Hierzu ist schematisch ein Steuergerät 21 dargestellt, das mittels Leitungen 22, 23 mit den aktiven, Wärme abgebenden Elementen der Pumpe, also dem Stator 19 und der Lagereinrichtung 20, verbunden ist und darüber die eingetragene elektrische und damit auch thermische Leistung ermitteln kann. Zudem ist das Steuergerät 21 mit dem ersten und zweiten Sensor 12, 13 verbunden, so dass es ebenfalls die Temperaturdifferenz zwischen Pumpeneinlass 3 und Pumpenauslass 4 erfassen kann. Das Steuergerät 21 kann einen Mikrocontroller umfassen, mittels dessen aus dem erfassten Temperaturunterschied und der eingetragenen Leistung unmittelbar der Blutdurchfluss ermittelt werden kann.

Innerhalb der Pumpe 1 können die Temperatursensoren 12, 13 beispielsweise im Bereich des Pumpeneinlasses 3 mittels eines Tragsterns im Fluidkanal 5 gehalten werden, oder ein Temperatursensor kann im Bereich des Vorleitrades 11 vor dem Rotor angeordnet und am Vorleitrad 11 befestigt sein. Ein Temperatursensor kann beispielsweise auch an der Innenwand des Gehäuses 2 im Bereich des Pumpeneinlasses befestigt sein.

Im Bereich des Pumpenauslasses 4 kann ein Temperatursensor ebenfalls im Auslasskanal mittels eines Tragsterns gehalten sein. Der Temperatursensor 13 kann jedoch auch an einer Wand des Pumpenauslasskanals 4 befestigt sein. Die Befestigung von Temperatursensoren an einem Tragelement, insbesondere an einem Tragstern, derart, dass das jeweilige Temperaturelement von der Wand des Pumpengehäuses 2 beabstandet ist, hat den Vorteil, dass die gesamte im Temperaturelement produzierte oder absorbierte Wärme mit der Flüssigkeit ausgetauscht wird, bevor eine Wärmeleitung zur Wand des Gehäuses 2 stattfindet.

Die oben beschriebenen Varianten zur Verwirklichung der Neuerung setzen voraus, dass eine Temperaturerfassung stromaufwärts und stromabwärts des Pumpenrotors oder eines Temperaturelements stattfindet.

Es ist jedoch auch möglich, die Durchflussrate der Flüssigkeit mittels eines einzigen thermischen Anemometrieeelements zu bestimmen, das in eine Pumpe integriert werden kann. Hierzu wird mit einem Element einerseits Wärme an die Flüssigkeit abgegeben oder aus der Flüssigkeit absorbiert und andererseits gleichzeitig eine Temperaturänderung des Temperaturelementes selbst bestimmt.

Hierzu kann das Temperaturelement mit einem Sensor verbunden sein, so dass beispielsweise ein Heizwiderstand mit einem Temperatursensor, beispielsweise einem Thermoelement, oder ein Peltier-Element mit einem Thermoelement gekoppelt ist, oder es kann sowohl die elektrische Energie in einem Heizwiderstand umgesetzt werden als auch die Temperatur des Heizwiderstandes durch die Temperaturabhängigkeit des elektrischen Widerstands gemessen werden. Auf diese Weise können sowohl der Energieeintrag als auch die Temperaturänderung mittels desselben Elementes gesteuert und erfasst werden.

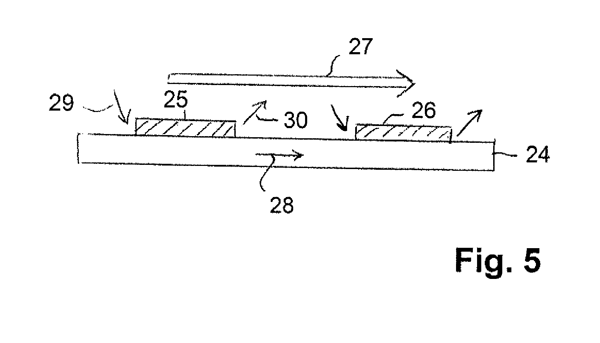

Hierzu ist in Figur 5 ein Element gezeigt, das nachfolgend erläutert wird. In Figur 5 ist im Schnitt ein metallisches Leiterelement 24 dargestellt, auf das Elektroden 25, 26 aufgebracht sind. Das Leiterelement 24 ist in einem Fluidstrom angeordnet, der durch den Pfeil 27 angedeutet ist, welcher die Strömungsrichtung zeigt. Fließt nun, wie durch den Pfeil 28 angedeutet, ein Strom durch den Leiter 24, so wird dieser je nach den Widerstandsgrößen wenigstens teilweise auch durch die Elektroden 25, 26 fließen, wobei in den Übergangsbereichen Thermoelemente gebildet sind, an denen eine Thermospannung durch die Wirkung des Stromes im Bereich des Materialübergangs entsteht. Mittels dieser Thermospannung, die gemessen werden kann, kann die Temperatur an dem Leiter 24 sehr genau bestimmt werden.

Zudem kann bei geeigneter Materialauswahl im Bereich der Materialübergänge zwischen den Elektroden 25, 26 und dem Leiter 24 der Peltiereffekt ausgenutzt werden, der beispielsweise im Bereich des Stromeintritts vom Leiter 24 in eine besser leitende Elektrode 25 eine Wärmeproduktion mit sich bringt (dargestellt durch den Pfeil 29, der einen Wärmeeintrag repräsentiert), während in dem Bereich, in dem der Strom von der Elektrode 25, 26 wieder in den Leiter 24 eintritt, eine Wärmeabsorption geschieht, dargestellt durch den Pfeil 30. Durch diese Effekte findet eine Temperaturänderung am Leiter 24 statt, die mittels des oben beschriebenen Thermoeffekts durch eine Spannungsänderung nachgewiesen werden kann.

Wird der Leiter, wie in dem gezeigten Beispiel, von einem Fluid umspült, so wird er durch das Fluid aktiv gekühlt. Die Temperaturänderungen bei einem bestimmten Stromdurchfluss hängen damit von der Flussrate des umspülenden Mediums ab. Mittels eines entsprechenden Kennlinienfeldes kann dann bei einem gegebenen Strom durch den Leiter 24 aus der erfassten Thermospannung die Durchflussrate der Flüssigkeit bzw. des Blutes bestimmt werden.

Die Verwendung eines Peltier-Elementes, bei dem sowohl die heizenden als auch die kühlenden Bereiche mit Blut einer Blutpumpe in Kontakt stehen, kann den Vorteil haben, dass durch gleichzeitige, örtlich verteilte Erwärmung und Kühlung insgesamt die Temperatur des Blutes weitgehend unverändert bleibt.

Es kann jedoch auch vorgesehen sein, dass bei Verwendung eines Peltier-Elementes nur ein Materialübergang mit dem Blut in Kontakt steht und das Peltier-Element mit wechselnden Stromrichtungen beaufschlagt wird, so dass der bestimmte Materialübergang abwechselnd Wärme abgibt und absorbiert. Dadurch wird dann das vorbeiströmende Blut ebenfalls abwechselnd aufgeheizt und abgekühlt, so dass insgesamt die im Körper eines Patienten fließende Blutmenge bezüglich der Temperatur nicht geändert wird.

Es kann dann bei demselben Element durch Erfassung der Thermospannung die Durchflussrate des Blutes bestimmt werden. Es ist jedoch auch denkbar, stromabwärts des Blutstroms die Temperaturschwankungen, die durch die abwechselnde Erwärmung und Abkühlung des Blutes stattfinden, zu erfassen und anhand der Ausschläge der Temperatur zu warmen und kühleren Temperaturen die Durchflussrate des Blutes zu bestimmen. Auch dies kann anhand einer Eichmessung und der Verwendung von entsprechenden Kennlinien geschehen.

Insgesamt lässt die Neuerung durch die Integration eines Durchflussmessers, der auf Thermodilutionsprinzipien beruht, in eine Pumpe eine platzsparende Lösung zu, wobei weitere Vorteile aus der Integration der Temperaturauswertung und der davon abgeleiteten Durchflussratenberechnung in die Steuereinrichtung für die Pumpe gezogen werden können.

**Patentansprüche**

1. Pumpe (1) zur Förderung von Flüssigkeiten, insbesondere Blutpumpe, mit einer Einrichtung zur Ermittlung der Durchflussrate, **gekennzeichnet durch** eine in die Pumpe integrierte Einrichtung (12, 13, 19, 20, 24, 25, 26) zur Zufuhr oder Ableitung von Wärmeenergie zur oder von der geförderten Flüssigkeit sowie eine in die Pumpe integrierte Einrichtung zur Ermittlung einer Temperatur, einer Temperaturänderung oder eines Temperaturgradienten.

2. Pumpe nach Anspruch 1, **dadurch gekennzeichnet, dass** eine Einrichtung zur Zufuhr von Wärmeenergie zu der geförderten Flüssigkeit durch einen in die Pumpe integrierten Antriebsmotor (8, 19) gebildet ist.

3. Pumpe nach Anspruch 1 oder 2, **dadurch gekennzeichnet, dass** eine Einrichtung zur Zufuhr von Wärmeenergie zu der geförderten Flüssigkeit durch ein oder mehrere in die Pumpe integrierte aktive, insbesondere magnetische Lager (20) gebildet ist.

4. Pumpe nach Anspruch 1, 2 oder 3, **dadurch gekennzeichnet, dass** im oder unmittelbar am Gehäuse (2) der Pumpe (1) ein im Strom der zu fördernden Flüssigkeit angeordnetes Heiz- oder Kühlelement (12, 13), insbesondere in Form eines Heizdrahtes oder eines Kühlelementes, insbesondere eines Peltier-Elementes, angeordnet ist.

5. Pumpe nach Anspruch 1, 2, 3 oder 4, **dadurch gekennzeichnet, dass** eine Einrichtung (13, 14) zur Ermittlung der Temperatur, einer Temperaturänderung oder eines Temperaturgradienten einen ersten und einen zweiten Temperatursensor (13, 14) umfasst, wobei einer der Temperatursensoren in der Strömung des zu fördernden Fluids stromaufwärts und der andere Temperatursensor stromabwärts der Einrichtung (12, 13, 19, 20, 24, 25, 26) zur Zufuhr oder Ableitung von Wärmeenergie angeordnet ist.

6. Pumpe nach Anspruch 5, **dadurch gekennzeichnet, dass** eine Einrichtung (12,13) zur Ermittlung der Temperatur, einer Temperaturänderung oder eines Temperaturgradienten einen ersten und einen zweiten Temperatursensor umfasst, wobei einer (12) der Temperatursensoren in der Strömung des zu fördernden Fluids stromaufwärts eines Pumpenrotors (8) und der andere Temperatursensor (13) stromabwärts des Pumpenrotors (8) angeordnet ist.

7. Pumpe nach Anspruch 4, **dadurch gekennzeichnet, dass** eine Einrichtung zur Ermittlung der Temperatur oder einer Temperaturänderung unmittelbar an dem Heiz- oder Kühlelement (12, 13, 24, 25, 26) angeordnet oder in dieses integriert ist.

8. Pumpe nach Anspruch 5, 6 oder 7, **dadurch gekennzeichnet, dass** eine Einrichtung (12, 13) zur Ermittlung einer Temperaturänderung unmittelbar an dem Heiz- oder Kühlelement angeordnet ist und dass eine Regelungseinrichtung (21) zur Einstellung einer Temperatur, einer Temperaturänderung oder eines Temperaturgradienten auf einen gegebenen Zielwert vorgesehen ist, wobei die zur Erreichung des Zielwertes zuzuführende Heiz- oder Kühlleistung ermittelt wird.

9. Pumpe nach Anspruch 1 oder einem der folgenden, **dadurch gekennzeichnet, dass** im Strom der zu fördernden Flüssigkeit ein Wärme abgebender Bereich (30) und/oder ein kühlender Bereich (29) eines Temperaturelementes (24, 25, 26) angeordnet ist und dass ein oder mehrere Temperatursensoren zur laufenden Temperaturerfassung des Elementes (24, 25, 26) und/oder der vorbeiströmenden Flüssigkeit vorgesehen sind.

10. Pumpe nach Anspruch 9, **dadurch gekennzeichnet, dass** zur Erfassung der Temperatur des Temperaturelementes ein temperaturabhängiger elektrischer Widerstand oder ein Thermoelement vorgesehen ist.

11. Verfahren zur Ermittlung der Flussrate einer zu fördernden Flüssigkeit durch eine Pumpe nach Anspruch 1, **dadurch gekennzeichnet, dass** die Flussrate unter Berücksichtigung einer erfassten Heiz- oder Kühlleistung, Temperatur, einer Temperaturdifferenz, einer elektrischen Motorleistung und insbesondere einer Drehzahl der Pumpe und einer elektrischen Lagerleistung ermittelt wird.

12. Verfahren zur Ermittlung der Flussrate einer zu fördernden Flüssigkeit durch eine Pumpe nach Anspruch 1, **dadurch gekennzeichnet, dass** ein mit der zu fördernden Flüssigkeit in Verbindung stehender Austauschbereich eines Temperaturelementes (24, 25, 26), insbesondere eines Peltier-Elementes, abwechselnd gekühlt und geheizt wird und dass die Temperatur des Austauschbereiches laufend gemessen und aus den Temperaturänderungen und/oder Heiz- oder Kühlleistung die Flussgeschwindigkeit bestimmt wird.

Fig. 1

Fig. 2

Fig. 3

Fig. 4

Fig. 5

Europäisches
Patentamt

European
Patent Office

Office européen
des brevets

**EUROPÄISCHER RECHERCHENBERICHT**

Nummer der Anmeldung

EP 15 18 3195

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/100414 A1 (TAMEZ DAN [US] ET AL) 10. April 2014 (2014-04-10) * For example, flow sensors which measure the rate of heat transfer to the flowing blood from a heated element can be employed; Absatz [0038] * ----- | 1-12 | INV. A61M1/10 A61M1/12 |
| X | WO 2014/141284 A2 (MAGENTA MEDICAL LTD [IL]) 18. September 2014 (2014-09-18) * thermal flow sensor * ----- | 1-12 | |
| X | WO 91/19170 A1 (MCPHERSONS LTD [AU]; HYBRID ELECTRONICS AUSTRALIA P [AU]) 12. Dezember 1991 (1991-12-12) * das ganze Dokument * ----- | 1-12 | |
| X | US 2009/306585 A1 (PANG CHANGLIN [US] ET AL) 10. Dezember 2009 (2009-12-10) * thermal flow sensor 144; Absätze [0061] - [0066]; Abbildungen 1-5 * ----- | 1-12 | |
| X | US 2014/073837 A1 (KERKHOFFS WOLFGANG [DE] ET AL) 13. März 2014 (2014-03-13) * Absätze [0032], [0034], [0092], [0093] * ----- | 1-12 | **RECHERCHIERTE SACHGEBIETE (IPC)** A61M G01F |

Der vorliegende Recherchenbericht wurde für alle Patentansprüche erstellt

| Recherchenort | Abschlußdatum der Recherche | Prüfer |
|---|---|---|
| Den Haag | 25. Februar 2016 | Van Veen, Jennifer |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

.......................................................
& : Mitglied der gleichen Patentfamilie, übereinstimmendes
   Dokument

EPO FORM 1503 03.82 (P04C03)

EP 3 135 327 A1

**ANHANG ZUM EUROPÄISCHEN RECHERCHENBERICHT
ÜBER DIE EUROPÄISCHE PATENTANMELDUNG NR.**

EP 15 18 3195

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-02-2016

| Im Recherchenbericht angeführtes Patentdokument | Datum der Veröffentlichung | Mitglied(er) der Patentfamilie | Datum der Veröffentlichung |
|---|---|---|---|
| US 2014100414 A1 | 10-04-2014 | AU 2013312527 A1 | 09-04-2015 |
| | | CA 2884180 A1 | 13-03-2014 |
| | | CN 104768589 A | 08-07-2015 |
| | | EP 2892583 A1 | 15-07-2015 |
| | | JP 2015527172 A | 17-09-2015 |
| | | KR 20150052215 A | 13-05-2015 |
| | | US 2014100414 A1 | 10-04-2014 |
| | | WO 2014039673 A1 | 13-03-2014 |
| WO 2014141284 A2 | 18-09-2014 | AU 2014229150 A1 | 24-09-2015 |
| | | CA 2905349 A1 | 18-09-2014 |
| | | EP 2967361 A2 | 20-01-2016 |
| | | US 2016022890 A1 | 28-01-2016 |
| | | US 2016051741 A1 | 25-02-2016 |
| | | WO 2014141284 A2 | 18-09-2014 |
| WO 9119170 A1 | 12-12-1991 | KEINE | |
| US 2009306585 A1 | 10-12-2009 | CA 2723724 A1 | 12-11-2009 |
| | | CN 102202719 A | 28-09-2011 |
| | | CN 104353150 A | 18-02-2015 |
| | | DK 2320989 T3 | 22-06-2015 |
| | | EP 2320989 A2 | 18-05-2011 |
| | | EP 2898911 A1 | 29-07-2015 |
| | | ES 2534864 T3 | 29-04-2015 |
| | | JP 5719767 B2 | 20-05-2015 |
| | | JP 2011519696 A | 14-07-2011 |
| | | JP 2015163192 A | 10-09-2015 |
| | | US 2009306585 A1 | 10-12-2009 |
| | | WO 2009137780 A2 | 12-11-2009 |
| US 2014073837 A1 | 13-03-2014 | EP 2895215 A2 | 22-07-2015 |
| | | US 2014073837 A1 | 13-03-2014 |
| | | WO 2014042925 A2 | 20-03-2014 |

EPO FORM P0461

Für nähere Einzelheiten zu diesem Anhang : siehe Amtsblatt des Europäischen Patentamts, Nr.12/82

12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4465063 A